# EUROPEAN PATENT SPECIFICATION

(11) **EP 0 636 119 B1**
(45) Date of publication and mention of the grant of the patent: **05.11.1997**
(21) Application number: 93905957.2
(22) Date of filing: 18.02.1993
(51) Int. Cl.: C07C 275/64, C07C 323/47, A61K 31/17

(54) **PHENYL SUBSTITUTED CYCLOALKYL HYDROXYUREA DERIVATIVES WHICH INHIBIT LIPOXYGENASE**
PHENYLSUBSTITUIERTE CYCLOALKYL-HYDROXYHARNSTOFFDERIVATE ALS LIPOXYGENASE-HEMMER
DERIVES D'HYDROXY-UREES CYCLOALKYLIQUES A SUBSTITUTION PHENYLIQUE INHIBANT LE LIPOXYGENASE

(30) Priority: 17.04.1992 JP 98150/92
(43) Date of publication of application: 01.02.1995
(73) Proprietor: PFIZER INC., New York, N.Y. 10017 (US)
(72) Inventor: NAKAO, Kazunari, Chita-gun, Aichi 470 (JP); KAWAI, Akiyoshi, Handa-shi, Aichi 475 (JP); STEVENS, Rodney W., Handa-shi, Aichi 475 (JP)
(74) Representative: Wood, David John
(86) International application number: US9301341
(87) International publication number: WO9321149

(56) References cited:
- EP-A- 0 436 199
- EP-A- 0 510 948
- WO-A-92/01682
- WO-A-92/09566
- WO-A-92/09567
- US-A- 5 187 192

## Description

This invention relates to novel phenylsubstituted cycloalkyl N-hydroxyurea derivatives. The compounds of the present invention inhibit the action of the enzyme lipoxygenase and are useful in the treatment or alleviation of inflammatory diseases, allergy and cardiovascular diseases in mammals. This invention also relates to pharmaceutical compositions comprising such compounds and to the use of such compounds in treating inflammatory diseases, allergy and cardiovascular diseases in mammals. This invention further relates to methods of making such compounds.

Arachidonic acid is known to be the biological precursor of several groups of endogenous metabolites, prostaglandins including prostacyclins, thromboxanes and leukotrienes. The first step of arachidonic acid metabolism is the release of arachidonic acid and related unsaturated fatty acids from membrane phospholipids, via the action of phospholipase. Free fatty acids are then metabolized either by cyclooxygenase to produce the prostaglandins and thromboxanes or by lipoxygenase to generate hydroperoxy fatty acids which may be further converted to the leukotrienes. Leukotrienes have been implicated in the pathophysiology of inflammatory diseases, including rheumatoid arthritis, gout, asthma, ischemia reperfusion injury, psoriasis and inflammatory bowel disease. Any drug that inhibits lipoxygenase is expected to provide significant new therapy for both acute and chronic inflammatory conditions.

Several review articles on lipoxygenase inhibitors have been reported (See H. Masamune et al., Ann. Rev. Med. Chem., **24**, 71-80 (1989) and B.J. Fitzsimmons et al., Leukotrienes and Lipoxygenases, 427-502 (1989).

Compounds of the same general class as the compounds of the present invention are disclosed in EP 279263 A2, EP 196184 A2, EP 0436199 Al, JP (Kohyo) 502179/88 and U.S. patent No. 4,822,809.

### SUMMARY OF THE INVENTION

The present invention provides ncvel phenylsubstituted cycloalkyl N-hydroxyurea derivatives of the following formula: 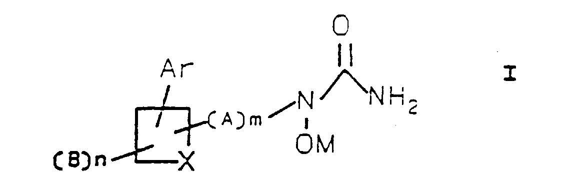 wherein
A is C₁ to C₄ alkylene or C₂ to C₆ alkenylene;
each B is independently halogen, C₁ to C₄ alkyl, C₂ to C₈ alkoxyalkyl or halosubstituted C₁ to C₄ alkyl;
X is methylene or ethylene;
M is hydrogen or a pharmaceutically acceptable cation;
Ar is mono-, di or tri-substituted phenyl wherein the substituents are independently selected from halogen, C₁ to C₄ alkyl, C₁ to C₄ alkoxy, halosubstituted C₁-C₄ alkyl, (C₁-C₄ alkyl)phenoxy, (C₁-C₄ alkoxy)phenoxy, halosubstituted phenoxy, halosubstituted (C₁ to C₄ alkyl)phenoxy, phenylthio, (C₁ to C₄ alkyl)phenylthio, (C₁ to C₄ alkoxy)phenylthio, halosubstituted phenylthio and halosubstituted (C₁ to C₄ alkyl)phenylthio; with the proviso that at least one of the substituents is phenylthio, (C₁ to C₄ alkyl)phenylthio, (C₁ to C₄ alkoxy)phenylthio, halosubstituted phenylthio or halosubstituted (C₁-C₄ alkyl)phenylthio;
A, B and Ar may be attached at any available position on the ring;
m is 0 or 1; and
n is 0, 1 or 2.
particularly preferred compounds of the invention are:-
N-hydroxy-N-(cis-3-[3(phenylthio)phenyl]cyclobutyl)urea;
N-hydroxy-N-(trans-3-[3-(phenylthio)phenyl]cyclobutyl)urea;
N-hydroxy-N-(cis-3-[3(4-methoxyphenylthio)phenyl]cyclobutyl)urea;
N-hydroxy-N-(trans-3-[3-(4-methoxyphenythio)phenyl]cyclobutyl)urea; and
N-(trans-3-[3-(4-fluorophenylthio)phenyl]cyclobutyl)N-hydroxyurea.

The compounds of formula I inhibit the enzyme 5-lipoxygenase. Therefore the compounds are useful for treating medical conditions for which a 5-lipoxygenase inhibitor is needed in a mammalian subject, e.g. a human subject. The compounds are especially useful for treating allergic and inflammatory conditions. This invention also embraces pharmaceutical compositions which comprise a compound of the formula I and a pharmaceutically acceptable carrier. This invention further relates to the use of the compounds for the manufacture of medicaments for treating inflammatory diseases, allergy and cardiovascular diseases in mammals.

### DETAILED DESCRIPTION OF THE INVENTION

### Definitions

As used herein, the following definitions are used.

"Halo" and "halogen" mean a radical derived from the elements fluorine, chlorine, bromine and iodine.

"Alkyl" means a straight or branched saturated hydrocarbon chain radical of 1 to 4 carbon atoms, for example, methyl, ethyl, n-propyl, isopropyl and n-butyl.

"Alkylene" means a straight or branched hydrocarbon chain spacer radical, for example, -CH₂-, -CH(CH₃)-, -CH₂CH₂- and -CH₂CH(CH₃)-.

"Alkenylene" means a straight or branched hydrocarbon chain spacer radical having one double bond, for example, -CH=CH-, -CH=CHCH₂- and -CH=CHCH(CH₃)-.

"Halosubstituted alkyl" refers to an alkyl radical as described above substituted with one or more halogen radicals, for example, chloromethyl, bromoethyl and trifluoromethyl.

"Alkoxy" means the group -OR wherein R is alkyl as defined above, fcr example, methoxy, ethoxy, propoxy, iscpropoxy and n-butoxy.

"Alkoxyalkyl" means the group -ROR' wherein R is alkylene as defined above and R' is alkyl as defined above, for example, methoxymethyl, ethoxymethyl, n-propoxymethyl, isopropoxymethyl, n-butoxymethyl, isobutoxymethyl and t-butoxymethyl.

"Pharmaceutically acceptable cation" means a non-toxic cation, for example, a cation based on the alkali and alkaline earth metals such as sodium, lithium, potassium and magnesium, as well as non-toxic ammonium, quatemary ammonium and amine cations such as ammonium, tetramethylammonium, tetraethylammonium, methylamine, diethylamine, trimethylamine and triethylamine.

The novel compounds of this invention may be prepared as shown in the reaction scheme described below.

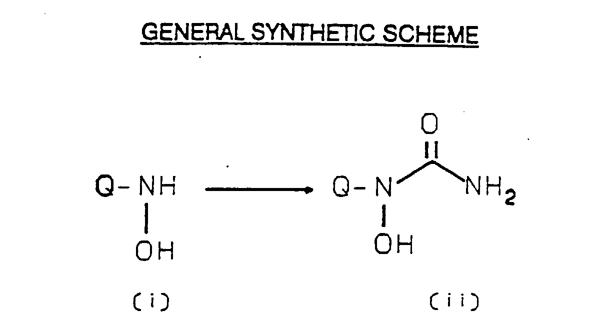

In the above scheme, Q is 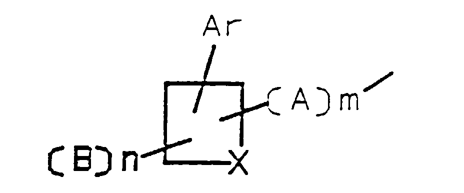 wherein Ar, B, X and n are as defined above.

Tne hydroxylamine (i) is treated with trimethylsilyl isocyanate (TMS-NCO) in a reaction-inert solvent usually at ambient through to reflux temperature. Suitable solvents which do not react with reactants and/or products include, for example, tetrahydrofuran (THF), dioxane, methylene chloride and benzene. An alternative prccedure employs treatment of (i) with gaseous hydrogen chloride in a reaction-inert solvent such as benzene or toluene and then subsequent treatment with phosgene. Reaction temperatures are usually in the range of ambient temperature through to boiling point of solvent. The intermediate carbamoyl chloride is not isolated but subjected to (i.e. in situ) reaction with aqueous ammonia. The product of formula (ii) thus obtained is isolated by standard methcds and purification can be achieved by conventional means, such as recrystallization and chromatography.

The aforementioned hydroxylamine (i) is easily prepared by standard synthetic procedures from readily available carbonyl compounds (i.e. ketones or aldehydes), alcohols or halogen compounds. For example, a suitable carbonyl compound is converted to its oxime and then reduced to the requisite hydroxylamine (i) with a suitable reducing agent. See, for example, R.F. Borch et al, J. Am. Chem. Soc., **93**, 2897 (1971). Reducing agents of choice include, but are not limited to, sodium cyanoborohydride and borane complexes such as boron-pyridine, boron-triethylamine and boron-dimethylsulfide, however triethylsilane in trifluoroacetic acid (TFA) may also be employed.

Alternatively, the hydroxylamine (i) can be prepared by treating the corresponding alcohol with N,O-bis(t-butoxycarbonyl)hydroxylamine under Mitsunobu-type reaction conditions followed by acid catalyzed hydrolysis of the N,O-protected intermediate product (see JP (Kokai) 45344/89).

The aforementioned hydroxylamine (i) may also be prepared from a suitable halide compound by the reaction with O-protected hydroxylamine and subsequent deprotection. See W.P. Jackson et al., J. Med. Chem., **31**, 499 (1988). Preferred O-protected hydroxylamines include, but are not limited to, O-tetrahydropyranyl-, O-trimethylsilyl- and O-benzylhydroxylamine. The hydroxylamine (i) thus obtained can be isolated by standard methods and purification can be achieved by conventional means, such as recrystallization and chromatography.

The individual isomers of the compounds of the invention can be prepared by a process which comprises separation employing a chiral stationary phase or kinetic enzymatic resoluticn cf an intermediate alcohol and transfcrmation of the optically active alcohol to the final compound by procedures as described above.

The pharmaceutically acceptable salts of the novel compounds of the invention are readily prepared by contacting said compounds with a stoichiometric amount of an appropriate metal hydroxide, alkoxide or amine in either aqueous solution or a suitable organic solvent. The respective salts may then be obtained by precipitation or by evaporation of the solvent.

The compounds of this invention inhibit the activity of the enzyme lipoxygenase. This inhibition has been demonstrated by an assay using rat peritoneal cavity-resident cells which determines the effect of said compounds on the metabolism of arachidonic acid, as described in "Synthesis of leukotrienes by peritoneal macrophages," Jap. J. Inflammation, **7**, 145-150 (1987). In this test some preferred compounds exhibited IC₅₀ values in the range from 0.01 to about 30 µM, for lipoxygenase inhibition.

The ability of the compounds of the present invention to inhibit lipoxygenase makes them useful for controlling the symptoms induced by the endogenous metabolites arising from arachidonic acid in a mammalian subject. Thus, the compounds are valuable in the prevention and treatment of such disease states in which the accumulation of arachidonic acid metabolites are the causative factor, e.g., allergic bronchial asthma, skin disorders, rheumatoid arthritis, osteoarthritis and thrombosis.

The compounds of the invention and their pharmaceutically acceptable salts are of particular use in the treatment or alleviation of inflammatory diseases in a human subject

### Methods of Administration

For treatment of the various conditions described above, the compounds of the invention and their pharmaceutically acceptable salts can be administered to a human subject either alcne or, preferably, in combinaticn with pharmaceutically acceptable carriers or diluents in a pharmaceutical composition, according to standard pharmaceutical practice. Tne compounds can be administered via a variety of conventional routes of administration including orally, parenterally and by inhalation. When the compounds are administered orally, the dose range will generally be from about 0.1 to about 20 mg/kg/day, based on the body weight of the subject to be treated, preferably from about 0.1 to about 1.0 mg/kg/day in single or divided doses. If parenteral administration is desired, then an effective dose will generally be from about 0.1 to about 1.0 mg/kg/day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically acceptable salts can be administered, for example, in the form of tablets, powders, lozenges, syrups or capsules, or as an aqueous solution or suspension. In the case of tablets for oral use, carriers which are commonly used include lactose and com starch. Lubricating agents such as magnesium stearate are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added.

For intramuscular, intraperitoneal, subcutaneous and intravenous use, a sterile solution of the active ingredient is usually prepared, and the pH of the solutions should be suitably adjusted and buffered. For intravenous use, the total concentration of solute should be controlled to make the preparation isotonic.

### Examples

Proton nuclear magnetic resonance spectra (NMR) were measured at 270 MHz unless otherwise indicated and peak positions are expressed in parts per million (ppm) downfield from tetramethylsilane. The peak shapes are denoted as follows: s, singlet; d, doublet; t, triplet; m, multiplet; br, broad.

Examples A and B are not part of the invention but illustrate the chemistry used in Examples 1 to 5 which illustrate the preparation of compounds of formula (I).

### Example A N-(trans-3-[3-(4-chlorophenoxy)phenyl]cyclobutyl)-N-hydroxyurea (11) and Example B, N-(cis-3-[3-(4-chlorophenoxylphenyl]cyclobutyl)-N-hydroxyurea (13)

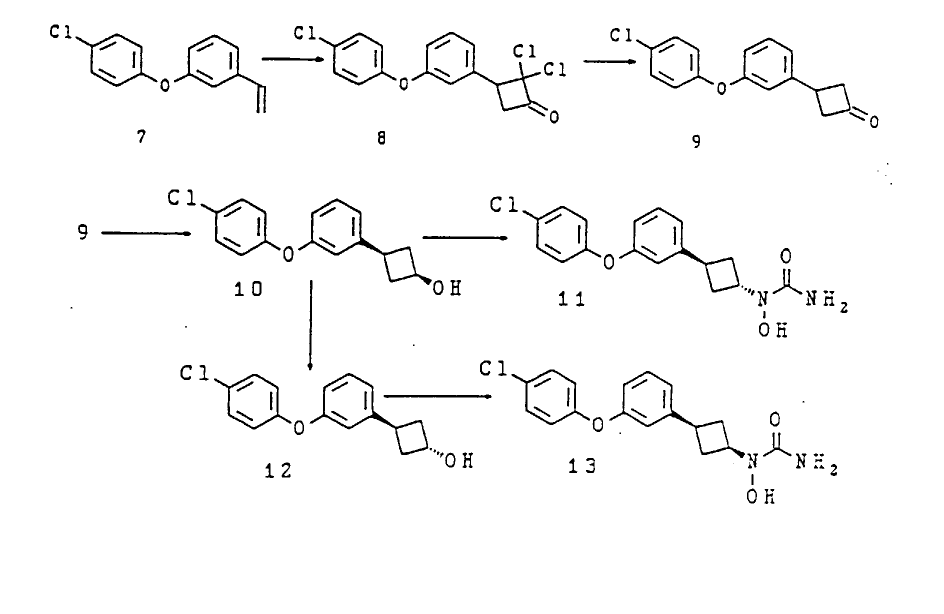

### Step 1, 3-(-4-chlorophenoxy)styrene (7)

The title compound was prepared from commercially avilable 3-(4-chlorophenoxy)benzaldehyde by Wittig methylenation (see J. Am. Chem. Soc., **104**, 4724 (1982) and J. Org. Chem., **52**, 3595 (1987)).

### Step 2, 3-[2-(4-chlorophenoxy)phenyl]cyclobutanone (9)

To a stirred solution of compound 7, from Step 1, above, (14.3 g, 62 mmol) and zinc-copper couple (4.46 g) in anhydrous ether (100 ml) was added dropwise a solution of trichloroacetyl chloride (7.27 ml, 65 mmol) and phosphoryl chloride (6.06 ml, 65 mmol) in anhydrous ether (45 ml) over a 1.5 hour period under a nitrogen atmosphere. When addition of the solution was complete, the mixture was refluxed with stirring for 2 hours. The reaction mixture was then filtered through a pad of Celite and the unreacted zinc-copper couple was washed with ether (100 ml). The ethereal solution was concentrated in vacuo to about 50% of its original volume, an equal volume (100 ml) of pentane was added and the solution stirred for a few minutes to precipitate the zinc salts. The solution was decanted from the residue, washed successively with ice water (100 ml), a cold saturated aqueous NaHCO₃ solution (100 ml), water (100 ml) and brine (100 ml), then was dried over MgSO₄ and the solvent was removed in vacuo to leave crude compound 8 (19 g) as a pale yellow oil which was used without further purification.

To a stirred solution of crude compound 8 (19 g) in acetic acid (60 ml) was added zinc powder (12 g) at about 10°C (exothermic). After stirring for 30 minutes at 50-70°C, the mixture was filtered through a pad of Celite and the unreacted zinc washed with acetic acid (20 ml) and ether (10 ml). The filtrate was concentrated in vacuo. Water (100 ml) was added to the mixture, and the whole was extracted with ether (2 x 100 ml, 50 ml). The organic layer was washed with saturated aqueous NaHCO₃ solution (100 ml), water (100 ml), brine (100 ml), dried over MgSO₄, and evaporated in vacuo to afford the title compound (9, 15.8 g, 93.5% yield from compound 7).

### Step 3, cis-3-[3-(4-chlorophenoxy)phenyl]cyclobutanol (10)

To a stirred solution of LiA[OC(CH₃)₃]₃H (17.7 g, 69.7 mmol) in THF (110 ml) cooled to -73°C was added compound 9, from Step 2, above, (58 mmol) in THF (30 ml) under a nitrogen atmosphere. The reaction mixture was stirred overnight at -70°C, and a cold saturated aqueous NH₄Cl (25 ml) was added. MgSO₄ (10 g) was added and the mixture filtered through Celite, washed with ethyl acetate (4 x 30 ml), and the filtrate was evaporated in vacuo to afford crude product. Ethyl acetate (100 ml) was added, the solution dried over MgSO₄ and concentrated in vacuo to provide the title compound (10, 10 g, 63% yield) as a colorless oil.

¹H NMR (CDCl₃)δ: 7.31-7.23 (m, 3H), 7.00-6.78 (m, 5H), 4.29-4.23 (m, 1H), 2.97-2.90 (m, 1 H), 2.80-2.70 (m, 2H), 2.04-1.92 (m, 2H), 1.83 (br s, 1H).

### Step 4, N-(trans-3-[3-(4-chloroohenoxyl)phenyl]cyclobutyl)-N-hydroxyurea (11)

To a stirred solution of compound 10, from Step 3, above, (3.08 g, 11.2 mmol), Ph₃P (2.61 g, 12.9 mmol) and N,O-bis(tert-butoxycarbonyl)hydroxylamine (3.01 g, 129 mmol) in THF (20 ml) was added DEAD (2.25 g, 129 mmol) in THF (5 ml) at room temperature under a nitrogen atmosphere. After stirring for 5 hours, volatiles were removed under reduced pressure. Diethylether (20 ml)/n-hexane (80 ml) was added to the resultant oil, insolubles were filtered off and the filtrate was concentrated in vacuo. The resulting oil was purified by flash chromatography (SiO₂ 100 g, n-hexane:ethylacetate = 30:1) to afford N,O-bis(tert-butoxycarbonyl)-N-trans-3-[3-(4-chlorophenoxy)phenyl]cyclobutyl)hydroxylamine (3.28 g, 60% yield).

To a stirred solution of N,O-bis(tert-butoxycarbonyl)-N-(trans-3-[3-(4-chlorophenoxy)phenyl]cyclobutyl)hydroxylamine (3.28 g, 6.7 mmol) in CH₂Cl₂ (30 ml) was added TFA (7 ml) dropwise at 5°C. After stirring for 3 hours, volatiles were removed in vacuo. saturated aqueous NaHCO₃ (70 ml) was added and the whole extracted with ethyl acetate (2 x 150 ml). The organic layer was washed with water (100 ml), brine (100 ml), dried over MgSO₄ and evaporated in vacuo to afford N-(trans-3-[3-(4-chlorophenoxy)phenyl]cyclobutyl)hydroxylamine (1.6 g).

To a stirred solution of N-(trans-3-[3(4-chlorophenoxy)phenyl]cyclobutyl)-hydroxylamine (1.6 g, 5.53 mmol) in THF (10 ml) was added TMS-NCO (0.87 g, 7.56 mmol) at room temperature under a nitrogen atmosphere. After stirring for 2 hours, methanol (10 ml) was added to quench the reaction. Volatiles were removed in vacuo, and the resulting solid was recrystallized from ethyl acetate/n-hexane to afford the Example 3 title compound (11, 0.94 g, 42.2% yield from protected hydroxylamine) as colorless needles, m.p. 132-134°C.

IR (nujol)ν: 3200, 1660, 1640, 1575, 1250, 1210, 830 cm⁻¹.
¹H NMR (DMSO-d₆)δ: 9.27 (s, 1H), 7.45-7.33 (m, 3H), 7.13 (d, J=7.7 Hz, 1H), 7.06-6.97 (m, 3H), 6.85-6.81 (m, 1H), 6.33 (s, 2H), 4.81-4.75 (m, 1H), 3.39-3.32 (m, 1H), 2.73-2.62 (m, 2H), 220·210 (m, 2H).

### Step 5, trans-3-[3-(4-chlorophenoxy)phenyl]cyclobutanol (12)

To a stirred solution of compound 10, from Step 3, above, (6.86 g, 25 mmol) and Ph₃P (5.82 g, 27.85 mmol) in THF (25 ml) was added benzoic acid (3.51 g, 28.75 mmol) in one portion, and then DEAD (5.01 g, 28.75 mmol) in THF (8 ml) was added dropwise at 10°C (exothermic) under a nitrogen atmosphere. After stirring for 1.5 hours at room temperature, the volatiles were evaporated in vacuo. Diethylether/n-hexane (20 ml/50 ml) was added to the residue, insolubles were removed by filtration and the filtrate was concentrated in vacuo. This treatment was repeated twice to provide crude trans-3-[2-(4-chlorophenoxy)phenyl]cyclobutyl benzoate (12 g) as a yellow oil, which was used for without further purification.

To a stirred solution of the crude trans-3-[3-(4-chlorophenoxy)phenyl]cyclobutyl benzoate (12 g) in THF/methanol (30 ml/30 ml) was added dropwise a KOH/water (27 g/45 ml) solution at room temperature. After stirring for 1.5 hours at room temperature, solvent was removed under reduced pressure and the whole was extracted with ethyl acetate (2 x 150 ml and 50 ml). The organic layer was washed with water (100 ml) and brine (200 ml), then was dried over MgSO₄ and evaporated in vacuo to afford the title compound (12, 5.62 g, 82% yield from compound 4).

¹H NMR (CDCl₃)_{δ}: 7.31-7.24 (m, 3H), 7.01-6.78 (m, 5H), 4.57-4.47 (m, 1H), 3.64-3.57 (m,1H), 2.51-2.34 (m, 4H), 1.70 (br s, 1H).

### Step 6, N-(cis-3-[3-(4-chlorophenoxy)phenyl]cyclobutyl)-N-hydroxyurea (13)

The Example 4 title compound (13, m.p. 152-153.5°C) was prepared from the corresponding trans-alcohol (12) by the same method as described in Step 4 of Example 3, above.

IR (nujol)ν: 3200, 1645, 1575, 1485, 1250, 1220, 945 cm⁻¹.
¹H NMR (DMSO-d₆)δ 9.17 (s, 1H), 7.43 (d, J=9.1 Hz, 2H), 7.33 (t, J=8 Hz, 1H), 7.06 6.81 (m, 5H), 6.29 (s, 2H), 4.66-4.60 (m, 1H), 3.11-3.04 (m, 1H), 2.41-2.23 (m, 4H).

### Example 1 N-hydroxy-N-(cis-3-[3-(phenylthio)phenyl]cyclobutyl)urea

The title compound was prepared from 3-phenylthiobenzaldehyde by methcds similar to those used in Examples A and B.
m.p.: 149-150°C.
IR (KBr)ν: 3500, 1640, 1570, 1450 cm⁻¹.
¹H NMR (DMSO-d₆)δ: 9.17 (s, 1H), 7.40-7.09 (m, 9H), 6.29 (s, 2H), 4.70-4.57 (m, 1H), 3.12-2.99 (m, 1H), 241-221 (m, 4H).

### Example 2 N-hydroxy-N-(trans-3-[3(phenylthio)phenyl]cyclobutyl)urea

The title compound was prepared from 3-phenylthiobenzaldehyde by methods similar to those used in Examples A and B.
m.p.: 118-119°C.
IR (KBr)ν: 3480, 1620, 1570, 1480, 1440 cm⁻¹.
¹H NMR (DMSO-d₆)δ: 9.27 (s, 1H), 7.45-7.26 (m, 8H), 7.12 (d, J=7.3 Hz, 1H), 6.33 (s, 2H), 4.80-4.73 (m, 1H), 3.45-3.34 (m, 1 H), 2.73-2.61 (m, 2H), 2.17-2.07 (m, 2H).

### Example 3 N-hydroxy-N-(cis-3-[3-'4-methoxyphenylthio)phenyl]cyclobutyl)urea

The title compound was prepared from 3-(4-methoxyphenylthio)benzaldehyde by methods similar to those used in Examples A and B.
m.p.: 153-154°C.
IR (KBr)ν: 3190, 1640, 1570, 1240 cm⁻¹.
¹H NMR (DMSO-d₆)δ: 9.16 (s, 1H), 7.41-7.37 (m, 2H), 7.26-7,19 (m, 1H), 7.06-6.98 (m, 4H), 6.89 (d, J=8.1 Hz, 1H), 6.28 (s, 2H), 4.65-4.58 (m, 1H), 3.78 (s, 3H), 3.04-2.97 (m, 1 H), 2.34-2.23 (m, 4H).

### Example 4 N-hydroxy-N-(trans-3-[3-(4-methoxyphenyithio)phenyl]cyclobutyl)urea

The title compound was prepared from 3-(4-methoxyphenylthio)benzaldehyde by methods similar to those used in Examples A and B.
m.p.: 124-125°C.
IR (KBr)ν: 3200, 1650, 1570, 1450, 1250 cm⁻¹.
¹H NMR (DMSO-d₆)δ: 9.25 (s, 1H), 7.44-7.38 (m, 2H), 7.25 (dd, J=7.7, 7.7 Hz, 1H), 7.15-6.98 (m, 4H), 6.91 (d, J=8.1 Hz, 1H), 6.32 (s, 2H), 4.77-4.71 (m, 1H), 3.78 (s, 3H), 3.40-3.22 (m, 1H), 270-259 (m, 2H), 2.14-206 (m, 2H).

### Example 5 N-(trans-3-[3-(4-fluorophenyithio)phenyl]cyclobutyl)-N-hydroxyurea

The title compound was prepared from 3-(4-fluorophenythio)benzaldehyde by methods similar to those used in Examples A and B.
m.p.: 135-136°C.
IR (KBr)ν: 3480, 1620, 1570, 1490 cm⁻¹.
¹H NMR (DMSO d₆)δ: 9.26 (s, 1H), 7.46-721 (m, 7H), 7.07 (br, J=7.69 Hz, 1H), 6.33 (s, 2H), 4.79-4.73 (m, 1H), 3.37-3.32 (m, 1H), 2.72-2.60 (m, 2H), 2.18-2.06 (m, 2H).

## Claims

1. A compound of the following chemical formula: 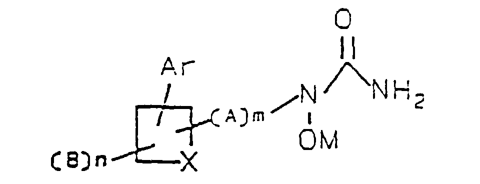 wherein
A is C₁-C₄ alkylene or C₂-C₆ alkenylene;
each B is independently halogen, C₁-C₄ alkyl, C₂-C₈ alkoxyalkyl or halosubstituted C₁-C₄ alkyl;
X is methylene or ethylene;
M is hydrogen or a pharmaceutically acceptable cation;
Ar is mono-, di- or tri- substituted phenyl wherein the substituents are each independently selected from halogen, C₁-C₄ alkyl, C₁-C₄ alkoxy, halosubstituted C₁-C₄ alkyl, (C₁-C₄ alkyl)phenoxy, (C₁-C₄ alkoxy)phenoxy, halosubstituted phenoxy, halosubstituted (C₁-C₄ alkyl)phenoxy, phenylthio, (C₁-C₄ alkyl)phenylthio, (C₁-C₄ alkoxy)phenylthio, halosubstituted phenylthio and halosubstituted (C₁-C₄ alkyl)phenylthio; with the proviso that at least one of the substituents is phenylthio, (C₁-C₄ alkyl)phenylthio, (C₁-C₄ alkoxy)phenylthio, halosubstituted phenylthio or halosubstituted (C₁-C₄ alkyl)phenylthio;
A, B and Ar may be attached at any available position on the ring;
m is 0 or 1; and
n is 0, 1 or 2.

2. A compound according to claim 1 wherein Ar is monosubstituted phenyl.

3. A compound according to claim 1 or 2 wherein m and n are each 0.

4. A compound according to claim 3 se!ected from:
N-hydroxy-N-(cis-3-[3-(phenyithio)phenyl]cyclobutyl)urea;
N-hydroxy-N-(trans-3-[3-(phenylthio)phenyl]cyclobutyl)urea;
N-hydroxy-N-(cis-3-(4-methoxyphenyithio)phenyl]cyclobutyl)urea;
N-hydroxy-N-(trans-3-[3-(4-methoxyphenylthio)phenyl]cyclobutyl urea; and
N-(trans-3-[3-(4-fluorophenylthio)phenyl]cyclobutyl)-N-hydroxyurea.

5. The use of a compound as claimed in any one of the preceding claims for the manufacture of a medicament for the treatment of a medical condition for which a 5-lipoxygenase inhibitor is needed.

6. The use according to claim 5, wherein the medical condition is an allergic or inflammatory condition.

7. A pharmaceutical composition for the treatment of an allergic or inflammatory condition in a mammalian subject which comprises a therapeutically effective amount of a compound of any one of claims 1 to 4 and a pharmaceutically acceptable carrier.

8. A compound as claimed in any one of claims 1 to 4 for use as a medicament.

## Patentansprüche

1. Verbindung der allgemeinen chemischen Formel 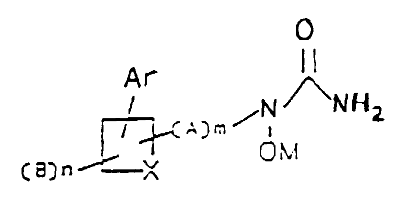 worin
A ein C₁-C₄-Alkylenrest oder C₂-C₆-Alkenylenrest ist;
jeder Rest B unabhängig Halogen, ein C₁-C₄-Alkyl-, C₂-C₈-Alkoxyalkyl- oder halogensubstituierter C₁-C₄-Alkylrest ist;
X ein Methylen- oder Ethylenrest ist;
M Wasserstoff oder ein pharmazeutisch annehmbares Kation ist;
Ar ein mono-, di- oder trisubstituierter Phenylrest ist, worin die Substituenten jeweils unabhängig ausgewählt sind aus Halogen, C₁-C₄-Alkyl-, C₁-C₄-Alkoxy-, halogensubstituierten C₁-C₄-Alkyl-, (C₁-C₄-Alkyl)phenoxy-, (C₁-C₄-Alkoxy) phenoxy-, halogensubstituierten Phenoxy-, halogensubstituierten (C₁-C₄-Alkyl)phenoxy-, Phenylthio-, (C₁-C₄ -Alkyl)phenylthio-, (C₁-C₄-Alkoxy)phenylthio-, halogensubstituierten Phenylthio- und halogensubstituierten (C₁-C₄-Alkyl)phenylthioresten; mit dem Vorbehalt, daß mindestens einer der Substituenten ein Phenylthio-, (C₁-C₄-Alkyl)phenylthio-, (C₁-C₄-Alkoxy)phenylthio-, halogensubstituierter Phenylthio- oder halogensubstituierter (C₁-C₄-Alkyl)phenylthiorest ist;
A, B und Ar an irgendeiner verfügbaren Position am Ring gebunden sein können;
m 0 oder 1 ist und
n 0, 1 oder 2 ist.

2. Verbindung nach Anspruch 1, worin Ar ein monosubstituierter Phenylrest ist.

3. Verbindung nach Anspruch 1 oder 2, worin m und n jeweils 0 sind.

4. Verbindung nach Anspruch 3, ausgewählt aus
N-Hydroxy-N-(cis-3-[3-(phenylthio)phenyl]cyclobutyl)harnstoff
N-Hydroxy-N-(trans-3-[3-(phenylthio)phenyl]cyclobutyl)harnstoff
N-Hydroxy-N-(cis-3-[3-(4-methoxyphenylthio)phenyl]cyclobutyl)harnstoff
N-Hydroxy-N-(trans-3-[3-(4-methoxyphenylthio)phenyl]cyclobutyl)harnstoff und
N-(trans-3-[3-(4-Fluorphenylthio)phenyl]cyclobutyl)-N-hydroxyharnstoff.

5. Verwendung einer Verbindung nach einem der vorhergehenden Ansprüche zur Herstellung eines Arzneimittels zur Behandlung eines medizinischen Zustandes, für den ein 5-Lipoxygenase-Inhibitor notwendig ist.

6. Verwendung nach Anspruch 5, worin der medizinische Zustand eine Allergie oder ein entzündlicher Zustand ist.

7. Pharmazeutische Zusammensetzung zur Behandlung eines allergischen oder entzündlichen Zustandes bei einem Säugetier umfassend eine therapeutisch wirksame Menge einer Verbindung nach einem der Ansprüche 1 bis 4 und einen pharmazeutisch annehmbaren Träger.

8. Verbindung nach einem der Ansprüche 1 bis 4 zur Verwendung als Arzneimittel.

## Revendications

1. Composé répondant à la formule chimique suivante : 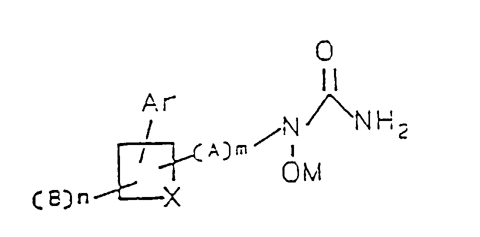 dans laquelle
A représente un groupe alkylène en C₁ à C₄ ou alcénylène en C₂ à C₆ ;
chaque symbole B représente indépendamment un halogène, un groupe alkyle en C₁ à C₄, alkoxyalkyle en C₂ à C₈ ou alkyle en C₁ à C₄ substitué avec un halogène ;
X représente un groupe méthylène ou éthylène ;
M représente l'hydrogène ou un cation pharmaceutiquement acceptable ;
Ar représente un groupe phényle mono-, di-, ou tri-substitué dont les substituants sont choisis chacun indépendamment entre des substituants halogéno, alkyle en C₁ à C₄, alkoxy en C₁ à C₄, alkyle en C₁ à C₄ substitué avec un halogène, (alkyle en C₁ à C₄)phénoxy, (alkoxy en C₁ à C₄)phénoxy, phénoxy substitué avec un halogène, (alkyle en C₁ à C₄)phénoxy substitué avec un halogène, phénylthio, (alkyle en C₁ à C₄)phénylthio, (alkoxy en C₁ à c₄)phényithio, phénylthio substitué avec un halogène et (alkyle en C₁ à C₄)phénylthio substitué avec un halogène ; sous réserve qu'au moins l'un des substituants consiste en un substituant phénylthio, (alkyle en C₁ à C₄)phénylthio, (alkoxy en C₁ à C₄)phénylthio, phénylthio substitué avec un halogène ou (alkyle en C₁ à C₄)phénylthio substitué avec un halogène ;
A, B et Ar peuvent être fixés à n'importe quelle position disponible sur le noyau ;
m est égal à 0 ou 1 ; et
n est égal à 0, 1 ou 2.

2. Composé suivant la revendication 1, dans lequel Ar représente un groupe phényle monosubstitué.

3. Composé suivant la revendication 1 ou 2, dans lequel m et n sont chacun égaux à 0.

4. Composé suivant la revendication 3, choisi entre :
la N-hydroxy-N-(cis-3-[3-(phénylthio)phényl]cyclobutyl)urée ; la N-hydroxy-N-(trans-3-[3-(phénylthio)phényl]cyclobutyl)-urée ;
la N-hydroxy-N-(cis-3-(4-méthoxyphénylthio)phényl]cyclobutyl)urée ;
la N-hydroxy-N-(trans-3-[3-(4-méthoxyphénylthio)phényl]cyclobutyl)urée ; et la N-(trans-3-[3-(4-fluorophénylthio)phényl]cyclobutyl)-N-hydroxyurée.

5. Utilisation d'un composé suivant l'une quelconque des revendications précédentes pour la production d'un médicament destiné au traitement d'un état médical pour lequel un inhibiteur de 5-lipoxygénase est requis.

6. Utilisation suivant la revendication 5, dans laquelle l'état médical est un état allergique ou inflammatoire.

7. Composition pharmaceutique pour le traitement d'un état allergique ou inflammatoire chez un mammifère, qui comprend une quantité thérapeutiquement efficace d'un composé suivant l'une quelconque des revendications 1 à 4 et un support pharmaceutiquement acceptable.

8. Composé suivant l'une quelconque des revendications 1 à 4, destiné à être utilisé comme médicament.
